# EUROPÄISCHE PATENTSCHRIFT

(11) **EP 0 642 784 B1**
(45) Veröffentlichungstag und Bekanntmachung des Hinweises auf die Patenterteilung: **19.05.1999**
(21) Anmeldenummer: 94113877.8
(22) Anmeldetag: 05.09.1994
(51) Int. Cl.: A61K 9/00

(54) **Brausemischung mit Alkalisalzen oder Lysinaten saurer, unlöslicher oder schwer löslicher Wirkstoffe**
Effervescent mixture containing the alkaline salts or lysinates of insoluble or hardly soluble acid drugs
Mélange effervescent contenant des sels alcalins ou lysinates de principes actifs acides insolubles ou à faible solubilité

(30) Priorität: 07.09.1993 CH 2652/93
(43) Veröffentlichungstag der Anmeldung: 15.03.1995
(60) Teilanmeldung: 98110087.8
(73) Patentinhaber: Gergely, Gerhard, Dr., A-1053 Wien (AT)
(72) Erfinder: Gergely, Gerhard, Dr., A - 1053 Wien (AT); Gergely, Thomas, Dr., A - 1053 Wien (AT); Gergely, Irmgard, A - 1053 Wien (AT); Gergely, Stefan, Dr., A - 1050 Wien (AT)
(74) Vertreter: Büchel, Kurt F., Dr.

(56) Entgegenhaltungen:
- EP-A- 0 369 228
- WO-A-93/00886

## Beschreibung

Die Erfindung betrifft Brausemischungen mit wenigstens einem sauren, unlöslichen oder schwerlöslichen pharmazeutischen Wirkstoff in Form seines löslichen Alkalisalzes oder Lysinates. Solche pharmazeutischen Wirkstoffe sind zum Beispiel organische Derivate von Essigsäure oder Propionsäure, z.B. Ibuprofen, Naproxen, Diclofenac, Butabarbital, Phenobarbital, Cefazolin, Diatrizoat, Ethacrynat, Flurbiprofen, Sulfacetamid oder Hetacillin.

Die sauren Wirkstoffe sind in aller Regel nicht fein dispers oder kolloidal erzielbar und werden deshalb gerne als ihre Kaliumsalze (z.B. Hetacillin) oder Lysinate (z.B. Ibuprofen), oder als ihre Natriumsalze (auch Ibuprofen, sowie die meisten anderen obenerwähnten Wirkstoffe) in Lösung verabreicht.

Die Alkalisalze solcher Wirkstoffe sind im Gegensatz zu den reinen Säuren im allgemeinen leicht wasserlöslich, haben allerdings oft einen bitteren Geschmack und fallen aus sauren Lösungen oft klumpig und/oder unter Schaumbildung aus; dasselbe passiert bei festen Arzneiformen durch die Magensäure. Diese Wirkstoffe führen daher leicht zu Irritationen der Magenwände.

Zur Vermeidung dieser Nachteile wird in der EP-A1-369 228 bereits vorgeschlagen, 100 Gewichtsteile eines wasserlöslichen Ibuprofen-Salzes mit 200 bis 1000 Gewichtsteilen Trägerstoff, 30 bis 80 Gewichtsteilen Stabilisator (z.B. Polyvinylpyrrolidon, "PVP") und 10 bis 100 Gewichtsteilen Alkalicarbonat zu granulieren, und das erhaltene Granulat mit 100 bis 400 Gewichtsteilen der Säurekomponente zu mischen. Ziel dabei ist es, das Natriumsalz nach dem Auflösen der Brausetablette in Lösung zu erhalten. Dies wird unter anderem dadurch erreicht, dass bei der gewählten Zusammensetzung der pH-Wert jedenfalls immer über 6 liegt. Eine solche Lösung schmeckt jedoch unangenehm, z.B. Diclofenac-Natrium sogar bitter. Ausserdem sind dazu enorme Mengen PVP nötig um zu verhindern, dass es während des Brausens zu einer Ausfällung des Wirkstoffes als Säure kommt.

Die Erfindung hat sich daher die Aufgabe gestellt, Brausemischungen der eingangs genannten Art zu schaffen, die eine verzögerte, immer aber fein disperse oder sogar kolloidale Ausfällung der Wirkstoffe unter Einwirkung saurer Lösungen oder der Magensäure ergeben. Dabei soll sich auch ein pH-Wert von 4 bis 5.5 einstellen, mit dem Brauselösungen bzw. -suspensionen wesentlich angenehmer zu trinken sind.

Diese Aufgabe wird erfindungsgemäss erstmals überraschend dadurch gelöst, dass sowohl das lösliche Wirkstoffsalz als auch die saure Komponente des Brausesystems oberflächenpassiviert sind. Bevorzugt umhüllt das Alkalisalz oder Lysinat die Partikeln der alkalischen Komponente des Brausesystems, z.B. Natriumbicarbonat und/oder Natriumcarbonat, und ist insbesondere mit weiterem Alkalicarbonat abgedeckt. Zweckmässig enthält die Wirkstoffschichte auch (auf 100 Gewichtsteile Wirkstoff) wenigstens 0,5, vorzugsweise 1 bis 3 Gewichtsteile eines Tensids, insbesondere Docusat-Natrium oder Natriumlaurylsulfat. Aus der Vielzahl von Rezeptmoglichkeiten auf der Suche nach der Lösung der gestellten Aufgabe die Kombination von sehr geringen (eventuell sogar gar keinen) Stabilisatormengen mit oberflächenpassivierten Komponenten auszuwählen, war selbst für den Fachmann nicht auf der Hand liegend und bedurfte einer Vielzahl von Überlegungsschritten.

Theoretisch kann man zwar schon einzelne Säuren mikronisiert erhalten. Die Ausfällung gemäss der vorliegenden Erfindung ist jedoch noch wesentlich feiner als ein feinpulveriger Wirkstoff erhältlich wäre. Dabei löst sich während des Auflösens der Brausetablette oder -mischung, durch die enge Anbindung an ein Natriumbicarbonat- oder ein Natriumcarbonat-Teilchen, zuerst das Alkalisalz oder Lysinat der Wirkstoffsäure; diese wird erst anschliessend in der für das Trinken zubereiteten Lösung durch die Säurekomponente des Brausesystems, beispielsweise innerhalb von 5 Sekunden, gegebenenfalls sogar kolloidal, jedenfalls aber fein dispers ausgefällt. Die feindispersen Teilchen bleiben aber längere Zeit suspendiert und ergeben daher eine angenehm zu trinkende Flüssigkeit.

In der erwähnten fein dispersen oder sogar kolloidalen Form wirkt die Säure nicht mehr aggressiv gegen die Magenwände, die ausserdem noch durch die Pufferwirkung des Brausesystems geschützt sind.

Bei den erfindungsgemässen Wirkstoffen handelt es sich meistens um solche, die nicht im sauren Milieu des Magens, sondern im alkalischen Milieu des Darms resorbiert werden sollen. Durch die fein disperse Form des Wirkstoffs in der Lösung ist auch die Magenpassage auf etwa 15 bis 20 Minuten beschränkt, so dass beim Eintritt in den Pylorus und den Darm durch die sehr hohe Oberfläche fein disperser, bzw. fast kolloidaler Säurepartikel des Wirkstoffes die sofortige Resorption erfolgen kann.

Um jedoch diesen Effekt erzielen zu können, dass das Wirkstoff-Alkalisalz oder Lysinat bei Auflösung der Brausetablette kurzzeitig in Lösung geht, und dass erst dann (verzögert) durch die sich bildende saure Lösung, die einen pH-Wert von 4 bis 5,5 haben kann, der Wirkstoff als Säure ausfällt, ist es nötig, das Wirkstoff-Alkalisalz oder -Lysinat sehr innig an das Alkalicarbonat, z.B. Natriumbicarbonat oder Natriumcarbonat, zu binden, damit um die Teilchen herum im Moment des Auflösens ein alkalischer Schutz erreicht wird, der die Ausfällung verzögert. Dies funktioniert nicht, wenn man das Wirkstoffsalz nur zu einer Brausebasis mischt, da hier im Moment des Auflösens sofort der Kontakt mit der Säure, z.B. Zitronensäure, gegeben ist.

Es ist daher zweckmässig, wenn man der Mischung noch pharmazeutisch bzw. toxikologisch unbedenkliche Tenside, wie z.B. Docusat-Natrium oder Natriumlaurylsulfat (dunkelfärbige oder pastöse, schlecht zu verarbeitende Tenside scheiden aus), vorzugsweise in Kombination mit Bindemitteln (wie z.B. Dextrin oder Polyvinylpyrrolidon oder Hydrokolloide, wie z.B. Maltodextrin) zusetzt. Dadurch werden die Suspensionseigenschaften der Wirkstoffsäure in der Flüssigkeit stark verbessert. Zu grosse Bindemittelmengen können allerdings unerwünschte Schaumbildung bewirken und die Auflösung der Brausetablette verzögern. Die Wirkung hängt auch davon ab, ob man mit wässriger oder alkoholischer Lösung arbeitet.

Daher ist es zweckmässig, wenn die Mischung zwar auf 100 Gewichtsteile Wirkstoff wenigstens 1, vorzugsweise 3 bis 5, jedoch insbesondere höchstens 20 Gewichtsteile eines Binde- bzw. Suspensionshilfsmittels, insbesondere Polyvinylpyrrolidon, enthält. Im Gegensatz zu der eingangs erwähnten EP-A1 sind hier nur geringe Bindemittelmengen vonnöten, da die Wirkstoffsäure aus ihrem Alkalisalz oder Lysinat bereits während des Auflösens der Brausetablette gebildet und feindispers in der Lösung verteilt werden soll. Deshalb wird bevorzugt auch ein Tensid eingesetzt, um beim Auflösen eine bessere Benetzung der Säureteilchen zu erzielen.

Die Bindung des Wirkstoffes kann man in verschiedener Weise erzielen:
1. Das Alkalisalz oder Lysinat wird gelöst, und die Lösung wird auf Natriumbicarbonat aufgezogen; anschliessend wird getrocknet; das Wirkstoffsalz kristallisiert an der Oberfläche des Natriumbicarbonatkristalls wieder aus.
2. Der feinst pulverisierte Wirkstoff wird mit dem Natriumbicarbonat gemischt; beide werden mit einem Bindemittel, z.B. Dextrin, agglomeriert. Auch damit kann man - da ja die Bindemittel verzögert löslich sind - eine Bindung des Wirkstoffsalzes an das Natriumbicarbonat oder Natriumcarbonat erreichen. Damit wird der Wirkstoff zuerst als Salz in Lösung gehen und erst zeitverzögert nach der Einwirkung und der Reaktion mit der Säurekomponente des Brausesystems feindispers bzw. kolloidal als Säure ausfallen.

### Beispiel 1:

25 Teile Diclofenac-Natrium werden mit 10 Teilen Propylenglykol und 40 Teilen Sorbit, sowie 1 Teil Polyvinylpyrrolidon K30 und 0,5 Teilen einer oberflächenaktiven Substanz, insbesondere Docusat-Natrium, in 40 Teilen Ethanol und 15 Teilen Wasser gelöst. Der Zusatz eines Tensids wirkt sich insbesondere dadurch vorteilhaft aus, dass im Stadium der Säurefällung die Partikel sofort benetzt werden und daher ein Zusammenklumpen noch besser hintangehalten wird. Diese Lösung wird auf 350 Teile Natriumbicarbonat aufgebracht und gut verteilt; anschliessend werden 100 Teile Natriumcarbonat zugegeben, das zuerst Wasser aufnimmt und erst anschliessend in Lösung geht, um den alkalischen Schutz zu verbessern. Die Masse wird dann im Vakuum getrocknet und auf die gewünschte Korngrösse von z.B. 0,1 bis 0,5 mm gesiebt. Das Granulat wird anschliessend zu einer sauren Brausebasis, bestehend aus einer festen, essbaren, organischen, oberflächenpassivierten Säure, zugemischt und gegebenenfalls zu Tabletten verpresst.

Die Oberflächenpassivierung kann in an sich bekannter Weise, z.B. wie in der EP-B1-272 312 oder WO93/00886 beschrieben, durchgeführt werden. Es ist aber auch möglich, die Umsetzung zwischen Säure und Carbonat bei der Passivierung zu umgehen, indem man die Oberfläche der Säurekristalle, z.B. der Zitronensäure, nensäure, mit einer Lösung von Natriumcitrat überzieht bzw. abdeckt und auf diese Schicht anschliessend wiederum Natriumbicarbonat und/oder Natriumcarbonat aus einer Lösung in Schichtform aufbringt. Dieses Aufbringen erfolgt besonders zweckmässig in einem Vakuummischkessel und gibt den bestmöglichen Schutz.

### Beispiel 2:

25 Teile Diclofenac-Natrium werden mit 350 Teilen Natriumbicarbonat vermischt und mit der folgenden Lösung zusammen granuliert: 15 Teile Dextrin, 1 Teil PVP und 0,5 Teile einer oberflächenaktiven Substanz werden in 20 Teilen Ethanol und 20 Teilen Wasser gelöst. Das Produkt wird anschliessend getrocknet, je nach Bedarf auf 0,1 bis 0,5 mm gesiebt und schliesslich wie in Beispiel 1 weiterbehandelt.

### Beispiel 3:

1 Gewichtsteil Docusat-Natrium, 2 Gewichtsteile PVP und 100 Gewichtsteile Naproxen-Natrium werden in 400 Gewichtsteilen 73%igem Alkohol gelöst. In einem Vakuummischer werden 700 Gewichtsteile Natriumbicarbonat unter Rühren auf 50°C erwärmt; die Lösung wird mittels Vakuum eingesaugt und unter Rühren homogen verteilt, worauf auf das nasse Granulat 150 Gewichtsteile Natriumcarbonat aufgebracht werden. Das Granulat wird abschliessend im Vakuum unter Rühren getrocknet und auf 0,25 mm gesiebt.

### Herstellung des mit Natriumbicarbonat oberflächenpassivierten Säuregranulates:

1400 Gewichtsteile kristalline und 200 Gewichtsteile pulverisierte Zitronensäure werden in einem Vakuummischer unter Rühren auf 60°C erwärmt und mit 6 Gewichtsteilen Wasser benetzt. Anschliessend werden 200 Gewichtsteile Natriumbicarbonat hinzugefügt und an der Zitronensäure-Oberfläche anreagiert, worauf anschliessend 50 Gewichtsteile Natriumcarbonat zugefügt werden. Das Produkt wird mittels Vakuum unter Rühren getrocknet. Das Naproxen- und das Säuregranulat werden miteinander ver

mischt und mit Süssstoff, Aroma sowie Füllstoffen versetzt. Man vermischt dann zur Homogenisierung und verpresst das Granulat zu Tabletten mit einem Tablettengewicht von 3,2 g.

Beim Einbringen der Brausetablette in Wasser löst sich die Tablette in ca. 80 sec auf, wobei das Naproxen als feine Suspension in der Lösung verteilt ist, bei einem pH-Wert von 4 - 4,5.

## Patentansprüche

1. Brausemischung mit einem aus einer alkalischen und einer sauren Komponente bestehenden Brausesystem, sowie wenigstens einem sauren, unlöslichen oder schwerlöslichen pharmazeutischen Wirkstoff in Form seines löslichen Alkalisalzes oder Lysinates, das in inniger Mischung mit der alkalischen Komponente des Brausesystems vorliegt, dadurch gekennzeichnet, dass sowohl das lösliche Wirkstoffsalz als auch die saure Komponente des Brausesystems oberflächenpassiviert sind.

2. Brausemischung nach Anspruch 1, dadurch gekennzeichnet, dass das Alkalisalz oder Lysinat - zweckmässig in Mischung mit einem Bindemittel - die Partikeln der alkalischen Komponente des Brausesystems, z.B. Natriumbicarbonat und/oder Natriumcarbonat, umhüllt und vorzugsweise mit weiterem Alkalicarbonat abgedeckt ist.

3. Brausemischung nach Anspruch 1 oder 2, dadurch gekennzeichnet, dass sie auf 100 Gewichtsteile Wirkstoff wenigstens 0,5, vorzugsweise 1 bis 3 Gewichtsteile eines Tensids, insbesondere Docusat-Natrium oder Natriumlaurylsulfat, enthält.

4. Brausemischung nach einem der vorhergehenden Ansprüche, dadurch gekennzeichnet, dass sie auf 100 Gewichtsteile Wirkstoff wenigstens 1, vorzugsweise 3 bis 5, und insbesondere höchstens 20 Gewichtsteile eines Binde- bzw. Suspensionshilfsmittels,insbesondere Polyvinylpyrrolidon, enthält.

## Claims

1. Effervescent mixture containing an effervescent system consisting of an alkaline and an acidic component, and at least one acidic, insoluble or hardly soluble pharmaceutical drug in the form of its soluble alkali metal salt or lysinate, which drug is present as an intimate mixture with the alkaline component of the effervescent system, characterized in that both the soluble drug salt and the acidic component of the effervescent system are passivated on the surface.

2. Effervescent mixture according to Claim 1, characterized in that the alkali metal salt or lysinate - expediently as a mixture with a binder - surrounds the particles of the alkaline component of the effervescent system, e.g. sodium bicarbonate and/or sodium carbonate, and is preferably covered with further alkali metal carbonate.

3. Effervescent mixture according to Claim 1 or 2, characterized in that it contains at least 0.5, preferably 1 to 3, parts by weight of a surfactant, in particular docusate sodium or sodium laurylsulphate, per 100 parts by weight of drug.

4. Effervescent mixture according to any of the preceding Claims, characterized in that it contains at least one, preferably 3 to 5, and in particular not more than 20, parts by weight of binder or suspending agent, in particular polyvinylpyrrolidone, per 100 parts by weight of drug.

## Revendications

1. Mélange effervescent avec un système effervescent constitué d'un composant alcalin et d'un composant acide, ainsi qu'au moins un principe actif pharmaceutique, acide, insoluble ou difficilement soluble, se présentant sous la forme de son sel alcalin soluble ou lysinate, se présentant en mélange intime avec le composant alcalin du système effervescent, caractérisé en ce que tant le sel de principe actif soluble, qu'également le composant acide du système effervescent sont passivés en surface.

2. Mélange effervescent selon la revendication 1, caractérisé en ce que le sel alcalin ou le lysinate - de manière appropriée en mélange avec un liant - enveloppe les particules du composant alcalin du système effervescent, par exemple du bicarbonate de sodium et/ou du carbonate de sodium, et, de préférence, est recouvert d'un autre carbonate alcalin.

3. Mélange effervescent selon la revendication 1 ou 2, caractérisé en ce qu'il contient, pour 100 parties en poids de principe actif, au moins 0,5 de préférence 1 à 3 parties en poids d'un tensioactif, en particulier du ducosate-sodium ou lauryl sulfate de sodium.

4. Mélange effervescent selon l'une des revendications précédentes, caractérisé en ce qu'il contient, pour 100 parties en poids de principe actif, au moins 1 de préférence 3 à 5, et en particulier au maximum 20 parties en poids d'un liant, respectivement d'un auxiliaire de suspension, en particulier du polyvinylpyrrolidon.
